Europäisches Patentamt

(19) European Patent Office

Office européen des brevets

(11) Publication number: **0 119 153**
**A2**

(12) # EUROPEAN PATENT APPLICATION

(21) Application number: **84710008.8**

(22) Date of filing: **08.03.84**

(51) Int. Cl.³: **A 61 K 39/12**
**A 61 K 37/02, C 07 C 103/52**

(30) Priority: **11.03.83 US 474690**

(43) Date of publication of application:
**19.09.84 Bulletin 84/38**

(84) Designated Contracting States:
**AT BE CH DE FR GB IT LI LU NL SE**

(71) Applicant: **THE RESEARCH FOUNDATION OF STATE UNIVERSITY OF NEW YORK**
**State University Plaza**
**Albany New York 12246(US)**

(72) Inventor: **Wimmer, Eckard**
**4 Hilltop Road**
**Stony Brook New York 11790(US)**

(72) Inventor: **Emini, Emilio A.**
**34 Peconic Street**
**Selden, New York 11784(US)**

(72) Inventor: **Jameson, Bradford A.**
**3 Ringneck Lane**
**East Setauket New York 11733(US)**

(74) Representative: **Prato, Roberto et al,**
**c/o Ingg. Carlo e Mario Torta Via Viotti 9**
**I-10121 Torino(IT)**

(54) Synthetic vaccines.

(57) A method is provided for the priming of physiological systems against infection by viruses which comprises administering to said system, prior to viral infection, an amino acid sequence selected from a hydrophilic region of the surface of the said virus. While the administration of said amino acid sequence may not cause the formation of neutralizing antibodies against said virus such neutralizing antibodies are formed upon the introduction of said virus into said system.

EP 0 119 153 A2

1

TITLE OF THE INVENTION

SYNTHETIC VACCINES

## BACKGROUND OF THE INVENTION

The problems which are inherent in vaccination with both live virus vaccines and attenuated virus vaccines are well known. While such vaccines have achieved enormous success these inherent problems have led many workers to study the possibility of manufacturing synthetic virus vaccines.

It has been shown that the surface portions of viruses, in particular, the amino acids which are designated as capsid peptides play a significant part in the process wherein the virus penetrates the host cell. Methods of determining which amino acids reside on the surface of the virus have been developed and furthermore, by computer analysis, it is possible to determine which of the surface polypeptide sequences are hydrophilic.

The current state of the art is well summarized in a recent article entitled Synthetic Vaccines by Richard A. Lerner (Scientific American, P. 66 January 1983). It has further been shown that viruses of the picorna group comprise four capsid polypeptides designated VP1, VP2, VP3 and VP4, of these, VP1 is generally believed to be the most important in the cell penetration steps. Lerner reports the selection of a 20 aminoacid peptide sequence (141 to 160) selected from the VP1 of foot and mouth disease virus, coupling same to a carrier molecule and, after determining that this peptide sequence elicited antibodies against itself proceeded to show that serum derived from the administration of such a peptide sequence did in fact serve to neutralize the virus when the virus was administered to an animal system together with said serum.

It is known, however, that certain viruses exist in very numerous modifications and that these modifications are often increased in number by mutation. The phenomenon is particularly well recognized in the case of Picorna viruses such as the Rhinovirus which is responsible, inter alia, for the common cold. The formation of synthetic vaccines to deal with each and every modification would be exceedingly tedious. Indeed, the lack of success in producing broad spectrum vaccines against influenza (a virus known for its propensity to mutate rapidly) is an indication of the difficulties of this approach. It is recognized of course that influenza virus does not fall in the category of the picorna viruses. Nevertheless it is an illustration of the general problem.

Thus, if a mode could be found of providing broad spectrum protection which would at least guard against initial contact, such a system would be a very valuable contribution to immuno-technology.

## SUMMARY OF THE INVENTION

It is our surprising finding that amino acid sequences selected from the capsid peptides of the viruses especially picorna viruses such as poliovirus while they do not, in themselves, form neutralizing antibodies against the virus and may therefore, in terms of classical immunological thinking be rejected as innocula, they would in fact serve to prime the system in such a way as to cause a immediate and high level of anti viral neutralizing antibodies to be produced upon the introduction of the virus into such a primed system.

The manner of selection of the peptides is similar to the approach taken in the Lerner monograph supra. A computer analysis is carried out upon the virus under investigation and the hydrophilic regions comprising the capsid peptides are designated. It is also possible by computer analysis to designate those sequences having high levels of hydrophilicity and therefore the highest probability of being involved in infection of the host cell.

The most hydrophilic sequences suitably of between 4 and 12 preferably between 6 and 8 amino acids are selected and synthesized. These sequences, optionally containing coupling zones comprising 2 or 3 additional amino acids such as TYR-GLY-GLY are then coupled to a carrier molecule. The use of a carrier protein is indicated since it is recognized that small amino acid sequences of the order discussed hereinabove themselves, are not usually recognized by immune systems. The predetermined amino acid sequence is coupled to such a carrier molecule and administered to the subject. It has been found that the carrier/short peptide sequence of 4-10 amino acids combination does not ellicit any neutralizing antibodies to the virus. Under the classical view therefore the administration of such material is quite useless. It has been our surprising finding, however, that if, subsequent to the administration of such a carrier/short peptide sequence the subject is exposed to what would normally be considered to be an infecting dose of the predetermined virus infection does not occur but a very substantial anti virus neutralizing antibody response is generated by the "infecting" organism. Thus, the first administration of the carrier/short pepetide sequence has a priming effect upon the system.

We have further found that where a slightly longer hydrophilic peptide sequence i.e. 10-12 amino acids is employed, not only will the priming effect occur but neutralizing antibody is also found.

## DESCRIPTION OF THE PREFERRED EMBODIMENTS

In the work disclosed herein a computer analysis (using the method of Hopp and Woods Proc. Nat. Acad. Sci (USA) 78: 3824, June (1981)) of the VP1 sequence of type 1 poliovirus led to the identification of several regions bearing continuous stretches of hydrophilic amino acid residues. Three of such six amino acid units were selected and arbitrarily designated peptide 1, peptide 2 and peptide 3. Peptide 1 comprises ARG - SER - ARG - SER - GLU - SER; peptide 2 comprises SER - THR - THR - ASN - LYS - ASP - LYS; and peptide 3 comprises ASP - ASN - PRO - ALA - SER - THR - THR - ASN - LYS - ASP - LYS.

Peptide 1 comprises the most hydrophilic area of VP1 and peptide 2 is a hydrophilic region which shows considerable variation when the sequences of the 3 polio virus serotypes are compared and Peptide 3 is peptide 2 extended by four additional adjacent amino acids. The peptides were synthesized by a modification of the method of Merrifield (Fed. Proc 21 412 (1962), J.Amer. Chem. Soc. 85 2149 (1963)). Spacer amino acids TYR - GLY - GLY were added similarly to the amino end and the peptides were then coupled to bovine serum album with bis diazotized benzidine in a basic medium, followed by purification by dialysis.

The carrier linked peptide is then administered to the subject at a level of between 0.1 and 1 suitably about 0.4 milligrams per kilogram of body weight. While priming is achieved by a single administration of the carrier/short peptide combination it is preferred to administer the priming material in three separate administrations suitably spaced between 1 and 4 weeks apart from each other. If desired a pharmacologically acceptable adjuvant may also be employed.

Where peptide 3 rather than peptide 1 or 2 was employed the presence of neutralizing antibody as well as priming was noted.

Peptide Synthesis (Modiciation of Merrifield Method).

Protected Amino Acids used for coupling tBOC = tertiary Butyloxycarbonyl

1) tBOC - Aspartic Acid (-0 Benzyl protection of carbonyl)

2) tBOC - Asparagine - ASN

3) tBOC - Arginine (-toluenesulfonyl) - ARG

4) tBOC - Alanine - ALA

5) tBOC - Cysteine (-methyl Benzyl) - CYS

6) tBOC - Glycine - GLU

7) tBOC - Glutamic (- 8 Benzyl) - GLU

8) tBOC - Glutamine - GLU

9) tBOC - Histidine (-toluenesulfonyl) - HIS

10) tBOC - Isoleucine - ILE

11) tBOC - Lysine (-ortho chlorobenzyloxycarbonyl)- LYS

12) tBOC - Leucine - LEU

13) tBOC - Methionine (sulfoxide) - MET

14) TBOC - Phenylalanine - PHE

15) TBOC - Proline - PRO

16) tBOC - Serine (benzyl) - SER

17) tBOC - Threonine (Benzyl) - THR

18) tBOC - Tyrosine (2,6-dichloro-benzyloxycarbonyl)-TYR

19) tBOC - Tryptophan (n-formyl) - TRY

20) tBOC - Valine - VAL

Solid Support: para Methylbenzyhdrylamine Resine
(.96 mM available amine.,
gm Resin

Repetitive Coupling - (3-5 equivalent of the appropriate amino acid used. (.8 gm Resin used)

The resin stored as the salt, therefore is first washed 1x1min, 2x5min w/5% Diisopropylethyl amine/Methylene Chloride, then washed 5x (1 min. each) with 35 mls Methylene Chloride. The amino acid to be coupled is then taken up in a minimal amount of Methylene Chloride and added to the Resin. This is equilibrated with the resin for 10 min. The coupling reaction is initiated with dicyclo-hexylcarbdiimide in an equal molar ratio with the amino acid. The coupling is allowed to proceed for 1-2 hours. The reaction mixture is then washed as follows: 5 x (1 min each) Methylene Chloride (35 ml/wash), 2x 1 min. Isoproparnol, 2x 1 min. Methylene chloride, 2x 1 min Isopropanol, 5x 1 min. Methylene chloride. At this point the reaction is assayed for coupling efficiency. If the efficiency of coupling is not ⩾ 99%, the coupling is repeated, such that each coupling reaction is ⩾ 99% efficient. The ∝ -amino group is then deprotected i.e. the tBOC group removed with 50% Trifluoroacetic acid/ Methylene Chloride [1x 1 min. 1 x 25 min.]. The Trifluoroacetic acid is then washed out: 5 x 1 min. Methylene Chloride 5 x 1 min Isopropanol, 5 x 1 min Methylene Chloride. The new generated ∝-amino group is then deprotonated: 1 x 1 min 5% diethylamine (DIEA) 2.5 min 5% DIEA. The resin is then washed 5 x 1 min Methylene Chloride in and the next coupling reaction may then be initiated.

Special Procedure for Glutamine and Asparagine

Dissolve 4 eq. of Boc-amino acid in a minimum volume of Dimethyl formamide. Add 4 eq. of 1-Hydroxy-benzotriazole. Cool to 0°C (ice-$H_2O$ both) let set for 10 min. Add to this mixture 4 eq. of Dicyclohexylcarbodiimide which has been previously chilled. Add this mixture to the reaction flask to initiate coupling.

(HF Apparatus: the entire apparatus is constructed of teflon.)

The dry peptide-resin and a teflon-coated stirring bar are placed in the reaction vessel. At least 50 equivalents of anisole per equivalent of peptide-resin are added. A Dewar Flask filled with either liquid nitrogen or dry ice/acetone is placed under and around the HF reservoir for 10 minutes. Teflon has a low thermal conductivity, and the inside of the vessel will require 10 minutes to become safely cold. The lines are evacuated but not long enough to distill the anisole. HF is distilled into the reservoir. The reservoir is warmed to room temperature, the reaction vessel is cooled with liquid nitrogen in a Dewar flask and the valve between the reaction vessel and the reservoir is slowly opened so that the HF gently boils. In the presence of anisole and HF, the peptide-resin turns red. When the desired amount of HF has distilled, approximately 10x excess over the anisole volume, all valves are closed and a stirred ice-water bath placed around the reaction vessel and stirred for 1 hr. When the reaction is complete stirring continues and the valve between the reaction vessel and the trap is <u>cautiously</u>
opened. The trap should be kept at -70°C. The HF
is distilled into the trap vessel. During the
entire time since opening the HF tank, th vacuum
pump has been isolated from the line. The system
should still be under pressure of only HF. After
all the HF has distilled from the trap, the line
to the vacuum pump is opened to effect complete
removal of the anisole. This may take 1-2 hours.
When the sample is dry, the resin-peptide mixture
is suspended in ether, stirred for 15 minutes at
room temperature. The resin and peptide to settle
are allowed, then the ether is poured off. Repeat
this 2-3 times. This will remove the residual anisole
and its oxidation products. After the ether washes,
the peptide should be dried and extracted in 5%
acetic acid. The peptides are then purified on
an Ion Exchange column, then a G-25 Sephadex Column.
In accordance with the above general procedures
and utilizing the appropriate tBOC protected amino
acids in the appropriate order there are produced
ARG - SER - ARG - SER - GLU - SER; TYR - GLY -
GLY - ARG - SER - ARG - SER - GLU - SER; SER -
THR - THR - ASN - LYS - ASP - LYS; TYR - GLY -
GLY - SER - THR - THR - ASN - LYS - ASP - LYS;
ASP - ASN - PRO - ALA - SER - THR - THR - ASN -
LYS - ASP - LYS; TYR - GLY - GLY - ASP - ASN -
PRO - ALA - SER - THR - THR - ASN - LYS --ASP -
LYS..

Conjugation of buffered Peptide #3 (TYR.-
GLY - GLY - ASP - ASN - PRO - ALA - SER - THR -
THR - ASN - LYS - ASP - LYS).

Linkage:

16.5 mg Peptide and 15.3 mg BSA (bovine serum albumin) are charged into a scintillation vial with a stir bar, an aqueous solution of saline sodium borate is added. (5.0 ml, 0.16 $\underline{M}$. Borate/.13 $\underline{M}$ NaCl pH 9.0) The mixture is stirred in an ice/$H_2O$ bath until the sample is completely dissolved and the pH is raised to 9.0 with 5 $\underline{N}$ sodium hydroxide. Bis-diazotized Benzidine is added dropwise (1 ml) to the reaction. The pH of the reaction is immediately adjusted to 9.0. Stirring is continued at 0°C for 2 hours. The product is dialyzed against $H_2O$ (48 hrs.) and then against 0.15 $\underline{M}$ NaCl (24 hrs.). The product may then be used as is or lyophilized and redissolved before use.

In accordance with the above procedure but using any of the other peptides produced in the previous example using a TYR - GLY - GLY spacer similar BSA coupled material is produced.

Similarly, similar products are obtained when physiologically acceptable carriers other than BSA are employed.

TABLE I

| Rabbit[a] | Inoculated Poliovirus Type | Weeks After Virus Inoculation | Plaque Titer Reduction[b] $(\log_{10}$ ppu/ml) | | | I.P. By Anti-serum[c] | | |
|---|---|---|---|---|---|---|---|---|
| | | | Type 1 | Type 2 | Type 3 | Type 1 | Type 2 | Type 3 |
| A | 1 | 1 | $>8.5$ | 1.4 | 1.0 | + | − | − |
| | | $\frac{3}{7}$ | $>8.5$ | 1.8 | $<1.0$ | + | − | − |
| B | 1 | 1 | $>8.5$ | 2.3 | 2.2 | + | − | + |
| | | 5 | $>8.5$ | 2.2 | $<1.0$ | + | + | + |
| | | 9 | $>8.5$ | 1.8 | $<1.0$ | + | + | + |
| C | 1 (Single Peptide Inoculation) | 1 | $>8.5$ | 2.6 | 1.7 | + | − | − |
| | | 5 | $>8.5$ | 2.1 | $<1.0$ | + | + | − |
| D | 1 (no peptide; singer virus inoculation) | 1 | $<1.0$ | $<1.0$ | $<1.0$ | − | − | − |
| E | 1 (control peptide) | 1 | $<1.0$ | $<1.0$ | $<1.0$ | − | − | − |
| F | 1 (hyperimmune) (+ Peptide) | 1 | $>8.5$ | 3.9 | $<1.0$ | + | + | − |
| | | 1 | $>8.5$ | $>6.7$ | $<1.0$ | + | + | − |
| G | 2 | 1 | 1.5 | 2.7 | $<1.0$ | − | | |
| | | 5 | $<1.0$ | 3.0 | $<1.0$ | − | + | − |
| H | 3 | 1 | $<1.0$ | $<1.0$ | $<1.0$ | − | − | + |
| I | 1,2 and 3 | 1 | $>8.5$ | $>6.7$ | $<1.0$ | + | + | − |
| | | 5 | 7.5 | $>6.7$ | $<1.0$ | + | + | − |

Table I: Priming of Anti-Poliovirus Immune Response by Peptide #1.

Footnotes:

[a]Rabbits A.B.G.H and I were inoculated as follows:

1.0 mg, per inoculation of BSA-linked peptide #1 (diluted 1:1 with complete Freund's Adjuvant; CFA) was inoculated into each rabbit by the I.D., S.C., and I.M. routes at 0.4, and 5 weeks, respectively. Approximately 25-75 ug of the appropriate poliovirus type(s) (1:1 with CFA) was inoculated, I.M., 10 days following the final peptide inoculation.

Rabbit E underwent an indentical inoculation schedule using a Rous Sarcoma virus sarc sequence-specific decapeptide (linked to Keyhole limpet hemocyanin as carrier protein) which bore no sequence homology with any poliovirus protein sequence.

Rabbit C received only the First I.D. inoculation of linked polio-specific peptide. 4 weeks later this was followed by inoculation, I.M. with virus.

Rabbit D received only a single inoculaton of I.M., 50 ug poliovirus type 1(1:1 with CFA).

Rabbit F was made hyperimmune to type 1 poliovirus by the inoculation of 50 mg of virus (1:1 with CFA) by the I.D., S.C., and I.M. routes at 0.4, and 5 weeks, respectively 10 days following the last virus inoculation, the rabbit received, I.M. 1.0 mg. BSA-linked peptide (1:1 with CFA).

[b]Plaque titer-reduction values were determined by a standard plaque reduction neutrlization test.

[c]Immunoprecipitation of [$^{35}$S] Met-labelled, purified virus by anti-serum using Staphylococcus aureus Protein A. This test measures the presence of an IgG response against the virus (Ey, et al. 1978).

| Rabbit[a] | Inoculated Poliovirus Type | Weeks After Virus inoculation | Plaque Titer Reduction[b] (log$_{10}$ ppu/ml) | | | I.P. by Anti-serum | | |
|---|---|---|---|---|---|---|---|---|
| | | | Type 1 | Type 2 | Type 3 | Type 1 | Type 2 | Type 3 |
| A | 1 | 1 | >8.5 | 3.6 | 3.3 | + | − | − |
| B | 1,2, and 3 | 1 | 5.5 | 3.2 | 1.4 | + | + | − |
| | | 5 | 6.3 | 2.7 | <1.0 | + | + | − |

[a] Both rabbits were inoculated with 1.0 mg.per inoculation of BSA-linked peptide #2 (Diluted 1:1 with complete Freund's Adjuvant; CFA) By the I.D., S.C., and I.M. routes at 0,4 and 5 weeks, respectively. Approximately 25-75 Mg of the appropriate poliovirus types(s) (1:1 with CFA) was inoculated, I.M., 10 days following the final peptide inoculation.

[b] Plaque titer-reduction values were determined by a standard plaque reduction neutralization test.

[c] Immunoprecipitation of [$^{35}$S] Met-labelled, purified virus by anti-serum using Staphylococcus Aureus Protein A. This test measures the presence of an IgG response against the virus (Ey, et al. 1978).

CLAIMS:

1)   Process of providing a material for priming an animal physiological system having the capability of immune response, to induce said system to produce neutralizing antibodies against a virus of a predetermined serotype upon introduction of such virus into said system characterized by comprising the sequential steps of:

determining the amino acid sequence on the surface of the virus,

selecting a region of hydrophilicity on the surface of said virus,

determining the amino acid sequence of said region,

selecting a sequence of 4-12 amino acids from said region,

and coupling a peptide containing said 4-12 amino acid sequence to a physiologically acceptable carrier large enough to be recognized by the immune mechanism of the system.

2)   A process in accordance with claim 1 characterized in that the virus is a Picorna virus.

3)   A process in accordance with claim 1 characterized in that the sequence is at least one among:
ARG - SER - ARG - SER - GLU - SER;
SER - THR - THR - ASN - LYS - ASP - LYS;
ASP - ASN - PRO - ALA - SER - THR - THR - ASN -
LYS - ASP - LYS.

4) A peptide characterized to be selected from the group consisting of:
ARG - SER - ARG - SER - GLU - SER; TYR - GLY - GLY - ARG - SER - ARG - SER - GLU - SER; SER - THR - THR - ASN - LYS - ASP - LYS; TYR - GLY - GLY - SER - THR - THR - ASN - LYS - ASP - LYS; ASP - ASN - PRO - ALA - SER - THR - THR - ASN - LYS - ASP - LYS; TYR - GLY - GLY - ASP - ASN - PRO - ALA - SER - THR - THR - ASN - LYS - ASP - LYS.

5) A material for priming an animal physiological system having the capability of immune response, to induce said system to produce neutralizing antibodies against a virus of a predetermined serotype upon introduction of such virus into said system characterized by comprising the carrier amino acid combination prepared in accordance with the process of claim 1.

6) A material in accordance with claim 5, characterized in that the virus is a Picorna virus.

7) A material in accordance with claim 5, characterized in that an amino acid spacer sequence is inserted between the selected sequence and the carrier.

8) A material for priming an animal physiological system having the capability of immune response, to induce said system to produce neutralizing antibodies against a virus of a predetermined serotype upon introduction of such virus into said system characterized by comprising a sequence of 4-12 amino acids from the region of hydrophilicity on the surface of said virus, coupled to a physiologically acceptable carrier large enough to be recognized by the immune mechanism of the system.

9) A material in accordance with claim 8, characterized in that the virus is a Picorna virus, optionally a polio virus.

10) A material in accordance with claim 9, characterized in that the sequence is selected from the appropriate VP1 peptide.

11) A material in accordance with claim 10, characterized in that an amino acid spacer sequence is inserted between the selected sequence and the carrier, and is optionally TYR - GLY - GLY.